(19) Europäisches Patentamt
European Patent Office
Office européen des brevets

(11) **EP 4 246 470 B1**

(12) **EUROPEAN PATENT SPECIFICATION**

(45) Date of publication and mention
of the grant of the patent:
**29.10.2025 Bulletin 2025/44**

(51) International Patent Classification (IPC):
***G06V 40/70*** *(2022.01)*

(52) Cooperative Patent Classification (CPC):
**G06V 40/70**

(21) Application number: **23161866.1**

(22) Date of filing: **14.03.2023**

(54) **SYSTEMS AND METHODS FOR CONTACTLESS ESTIMATION OF WRIST SIZE**

SYSTEME UND VERFAHREN ZUR KONTAKTLOSEN SCHÄTZUNG DER HANDGELENKSGRÖSSE

SYSTÈMES ET PROCÉDÉS D'ESTIMATION SANS CONTACT DE LA TAILLE DU POIGNET

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **15.03.2022 US 202263319861 P**
**06.03.2023 US 202318178643**

(43) Date of publication of application:
**20.09.2023 Bulletin 2023/38**

(73) Proprietor: **Perfect Mobile Corp.**
**New Taipei City 231 (TW)**

(72) Inventors:
• **CHENG, Chih-Yu**
**116 Taipei City (TW)**
• **PENG, Hsin-Yi**
**831 Kaohsiung City (TW)**
• **CHANG, Hua-Jen**
**231 New Taipei City (TW)**

(74) Representative: **Zinkler, Franz**
**Schoppe, Zimmermann, Stöckeler**
**Zinkler, Schenk & Partner mbB**
**Patentanwälte**
**Radlkoferstrasse 2**
**81373 München (DE)**

(56) References cited:
• **MICHAEL J REALE ET AL: "A Multi-Gesture Interaction System Using a 3-D Iris Disk Model for Gaze Estimation and an Active Appearance Model for 3-D Hand Pointing", IEEE TRANSACTIONS ON MULTIMEDIA, IEEE, USA, vol. 13, no. 3, 1 June 2011 (2011-06-01), pages 474 - 486, XP011322965, ISSN: 1520-9210, DOI: 10.1109/TMM.2011.2120600**

## Description

### TECHNICAL FIELD

The present disclosure generally relates to systems and methods for contactless estimation of wrist size.

It is an object of the present invention to provide systems, methods and non-transitory storage media for the contactless estimation of wrist size. This object is achieved by a method of any one of claims 1, 5, 15, a system of any one of the claims 7, 17, 18, or a non-transitory storage medium of any one of claims 11, 19, 20.

An example of a prior art document which teaches multi-gesture interaction by computing different biometric measures from the eyes and the hand is Michael J Reale et al.: "A Multi-Gesture Interaction System Using a 3-D Iris Disk Model for Gaze Estimation and an Active Appearance Model for 3-D Hand Pointing", IEEE Transactions on Multimedia, IEEE, USA, vol. 13, no. 3, 1 June 2011 (2011-06-01), pages 474-486, XP011322965, ISSN: 1520-9210, DOI: 10.1109/TMM.2011.2120600.

### SUMMARY

In accordance with one embodiment, a computing device prompts a user to place the user's hand and wrist next to a facial region of the user. The computing device obtains an image depicting the facial region and the wrist of the user. The computing device measures a diameter of an iris in the facial region depicted in the image and measures a width of the wrist of the hand depicted in the image. The computing device estimates an actual wrist width of the user's hand based on an actual iris diameter, the measured diameter of the iris in the facial region depicted in the image, and the measured width of the wrist of the hand in the image.

In accordance with another embodiment, a computing device prompts a user to place the user's hand and wrist next to a facial region of the user. The computing device obtains an image depicting the facial region and a wrist of the user. The computing device measures a pupillary distance between eyes depicted of the image and measures a width of a wrist of the hand depicted in the image. The computing device estimates an actual wrist width of the user's hand based on an actual pupillary distance, the measured wrist width of the hand depicted in the image, and the measured pupillary distance of the hand depicted in the image.

Another embodiment is a system that comprises a memory storing instructions and a processor coupled to the memory. The processor is configured by the instructions to prompt a user to place the user's hand and wrist next to a facial region of the user. The processor is further configured to obtain an image depicting the facial region and the wrist of the user. The processor is further configured to measure a diameter of an iris in the facial region depicted in the image and measure a width of the wrist of the hand depicted in the image. The processor is further configured to estimate an actual wrist width of the user's hand based on an actual iris diameter, the measured diameter of the iris in the facial region depicted in the image, and the measured width of the wrist of the hand in the image.

Another embodiment is a non-transitory computer-readable storage medium storing instructions to be implemented by a computing device. The computing device comprises a processor, wherein the instructions, when executed by the processor, cause the computing device to prompt a user to place the user's hand and wrist next to a facial region of the user. The processor is further configured by the instructions to obtain an image depicting the facial region and the wrist of the user. The processor is further configured by the instructions to measure a diameter of an iris in the facial region depicted in the image and measure a width of the wrist of the hand depicted in the image. The processor is further configured by the instructions to estimate an actual wrist width of the user's hand based on an actual iris diameter, the measured diameter of the iris in the facial region depicted in the image, and the measured width of the wrist of the hand in the image.

In accordance with another embodiment, a computing device prompts a user to place the user's hand next to a facial region of the user with the hand being held in a first roll orientation and then with the hand being held in a second roll orientation. The computing device obtains a first image at the first roll orientation and a second image at the second roll orientation, the first image and the second image each depicting the facial region and a wrist of the user. The computing device determines a first wrist rotation angle ($\theta_1$) at the first roll orientation in the first image and a second wrist rotation angle ($\theta_2$) at the second roll orientation in the second image. The computing device measures a first diameter of an iris in the facial region at the first roll orientation in the first image and a second diameter of the iris in the facial region at the second roll orientation in the second image. The computing device measures a first width of a wrist of the hand at the first roll orientation in the first image and a second width of the wrist of the hand at the second roll orientation in the second image. The computing device estimates an actual wrist width of the hand based on the first measured wrist width, the second measured wrist width, the first wrist rotation angle ($\theta_1$), and the second wrist rotation angle ($\theta_2$).

Other systems, methods, features, and advantages of the present disclosure will be apparent to one skilled in the art upon examining the following drawings and detailed description.

## BRIEF DESCRIPTION OF THE DRAWINGS

Various aspects of the disclosure are better understood with reference to the following drawings. The components in the drawings are not necessarily to scale, with emphasis instead being placed upon clearly illustrating the principles of the present disclosure. Moreover, in the drawings, like reference numerals designate corresponding parts throughout the several views.

FIG. 1 is a block diagram of a computing device configured to perform contactless wrist size estimation according to various embodiments of the present disclosure.

FIG. 2 is a schematic diagram of the computing device of FIG. 1 in accordance with various embodiments of the present disclosure.

FIG. 3 is a top-level flowchart illustrating examples of functionality implemented as portions of the computing device of FIG. 1 for performing contactless wrist size estimation according to various embodiments of the present disclosure.

FIG. 4 is a top-level flowchart illustrating examples of functionality implemented as portions of the computing device of FIG. 1 for performing contactless wrist size estimation according to alternative embodiments of the present disclosure

FIG. 5 illustrates an example user interface generated by the computing device of FIG. 1 according to various embodiments of the present disclosure.

FIG. 6 illustrates an example user interface for guiding the user regarding hand placement according to various embodiments of the present disclosure.

FIG. 7 illustrates the computing device of FIG. 1 estimating the actual wrist size of the user based on the measured iris diameter and the measured wrist width of the user according to various embodiments of the present disclosure.

FIG. 8 illustrates the computing device of FIG. 1 estimating the actual wrist size of the user based on the measured iris diameter and the measured pupillary distance of the user according to various embodiments of the present disclosure.

FIG. 9 illustrates the user's hand being held at two different wrist rotation angles according to various embodiments of the present disclosure.

FIG. 10 is a top-level flowchart illustrating examples of functionality implemented as portions of the computing device of FIG. 1 for performing contactless wrist size estimation according to alternative embodiments of the present disclosure.

## DETAILED DESCRIPTION

The subject disclosure is now described with reference to the drawings, where like reference numerals are used to refer to like elements throughout the following description. Other aspects, advantages, and novel features of the disclosed subject matter will become apparent from the following detailed description and corresponding drawings.

There is a need for an improved way for allowing consumers to determine a wrist size without the use of a wrist-sizing tool since not everyone has access to a tape measure to measure their wrist size. The present disclosure is directed to systems and methods for allowing users to hold up their hand in close proximity to their face, where the system captures an image of the user, measures the iris diameter, the pupillary distance, and wrist width of the user, and estimates the user's actual wrist size by using one or more of the measured values in combination with the actual iris diameter value or actual pupillary distance value.

For purposes of this disclosure, the actual wrist size generally refers to the actual wrist circumference measured around the wrist. Estimating the actual wrist size allows a user to find the proper size for an accessory such as a watch or a bracelet. Furthermore, for purposes of this disclosure, a value of 11.7 mm is used for the actual iris diameter because the average white-to-white horizontal diameter of the eye is approximately 11.7 mm $\pm$ 0.5 mm. Exemplary embodiments utilize 11.7 mm as a reference value when estimating the wrist size of the user. The pupillary distance is the distance between the centers of the user's pupils. The system outputs the estimated actual wrist size or estimated actual wrist circumference of the user.

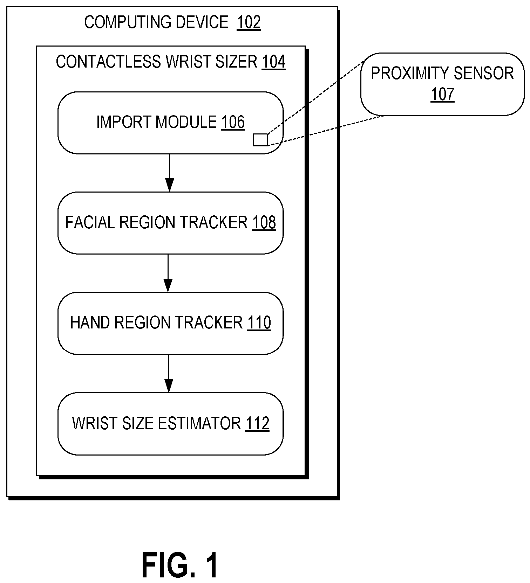

A description of a system for implementing contactless wrist size estimation is described followed by a discussion of the operation of the components within the system. FIG. 1 is a block diagram of a computing device 102 in which the embodiments disclosed herein may be implemented. The computing device 102 may comprise one or more processors that execute machine executable instructions to perform the features described herein. For example, the computing device 102 may be embodied as a computing device such as, but not limited to, a smartphone, a tablet-computing device, a laptop, and so on.

A contactless wrist sizer 104 executes on a processor of the computing device 102 and includes an import module 106, a facial region tracker 108, a hand region tracker 110, and a wrist size estimator 112. The import module 106 is configured to obtain digital images of a user holding the user's hand next to the user's face. For some embodiments, the import module 106 guides the user regarding optimal placement of the user's hand next to the user's face. A proximity sensor 107 in the import module 106 detects when the user's hand is within a threshold distance of the user's face since placement of the user's hand close to the user's face generally allows the computing device 102 to perform a more accurate estimation of the user's wrist size.

When the proximity sensor 107 determines that the user's hand is within a threshold distance of the user's face, the import module 106 obtains a digital image of the user. For some embodiments, the import module 106 is configured to cause a camera (e.g., front-facing camera) of the computing device 102 to capture an image or a video of a user of the computing device 102. Alternatively, the import module 106 may obtain an image or video of the user from another device or server where the computing device 102 may be equipped with the capability to connect to the Internet.

The images obtained by the import module 106 may be encoded in any of a number of formats including, but not limited to, JPEG (Joint Photographic Experts Group) files, TIFF (Tagged Image File Format) files, PNG (Portable Network Graphics) files, GIF (Graphics Interchange Format) files, BMP (bitmap) files or any number of other digital formats. The video may be encoded in formats including, but not limited to, Motion Picture Experts Group (MPEG)-1, MPEG-2, MPEG-4, H.264, Third Generation Partnership Project (3GPP), 3GPP-2, Standard-Definition Video (SD-Video), High-Definition Video (HD-Video), Digital Versatile Disc (DVD) multimedia, Video Compact Disc (VCD) multimedia, High-Definition Digital Versatile Disc (HD-DVD) multimedia, Digital Television Video / High-definition Digital Television (DTV/HDTV) multimedia, Audio Video Interleave (AVI), Digital Video (DV), QuickTime (QT) file, Windows Media Video (WMV), Advanced System Format (ASF), Real Media (RM), Flash Media (FLV), an MPEG Audio Layer III (MP3), an MPEG Audio Layer II (MP2), Waveform Audio Format (WAV), Windows Media Audio (WMA), 360 degree video, 3D scan model, or any number of other digital formats.

The facial region tracker 108 is configured to track the eyes of the user depicted in the captured image and measure a diameter of an iris in the facial region. For some embodiments, the facial region tracker 108 measures the diameter of the iris of the user's eye closest to the user's hand depicted in the captured image. For some embodiments, the facial region tracker 108 is configured to also measure a pupillary distance between the user's eyes depicted in the image.

The hand region tracker 110 is configured to track a hand of the user depicted in the image and measure the width of a wrist region on the hand depicted in the image. The wrist size estimator 112 is configured to estimate a wrist size of the wrist based on an actual iris diameter, an actual pupillary distance, the measured iris diameter, the measured wrist width, the measured pupillary distance, or any combination thereof. The wrist size estimator 112 then outputs the estimated wrist size.

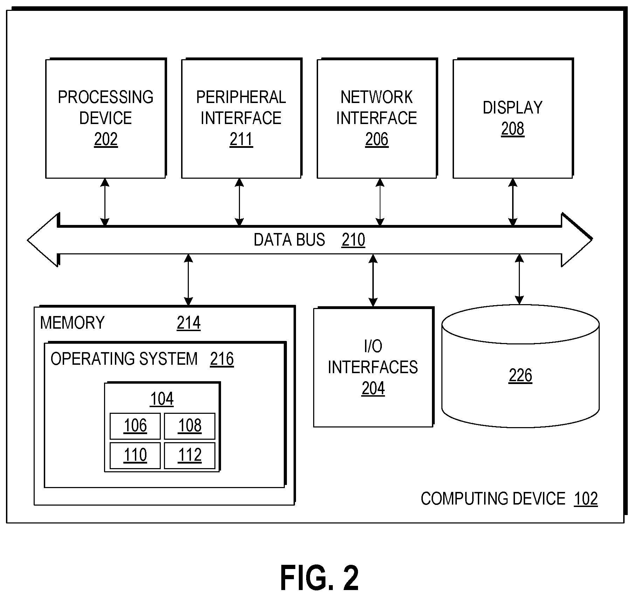

FIG. 2 illustrates a schematic block diagram of the computing device 102 in FIG. 1. The computing device 102 may be embodied as a desktop computer, portable computer, dedicated server computer, multiprocessor computing device, smart phone, tablet, and so forth. As shown in FIG. 2, the computing device 102 comprises memory 214, a processing device 202, a number of input/output interfaces 204, a network interface 206, a display 208, a peripheral interface 211, and mass storage 226, wherein each of these components are connected across a local data bus 210.

The processing device 202 may include a custom made processor, a central processing unit (CPU), or an auxiliary processor among several processors associated with the computing device 102, a semiconductor based microprocessor (in the form of a microchip), a macroprocessor, one or more application specific integrated circuits (ASICs), a plurality of suitably configured digital logic gates, and so forth.

The memory 214 may include one or a combination of volatile memory elements *(e.g.,* random-access memory (RAM, such as DRAM, and SRAM, *etc.))* and nonvolatile memory elements *(e.g.,* ROM, hard drive, tape, CDROM, *etc.).* The memory 214 typically comprises a native operating system 216, one or more native applications, emulation systems, or emulated applications for any of a variety of operating systems and/or emulated hardware platforms, emulated operating systems, *etc.* For example, the applications may include application specific software that may comprise some or all the components of the computing device 102 displayed in FIG. 1.

In accordance with such embodiments, the components are stored in memory 214 and executed by the processing device 202, thereby causing the processing device 202 to perform the operations/functions disclosed herein. For some embodiments, the components in the computing device 102 may be implemented by hardware and/or software.

Input/output interfaces 204 provide interfaces for the input and output of data. For example, where the computing device 102 comprises a personal computer, these components may interface with one or more input/output interfaces 204, which may comprise a keyboard or a mouse, as shown in FIG. 2. The display 208 may comprise a computer monitor, a plasma screen for a PC, a liquid crystal display (LCD) on a hand held device, a touchscreen, or other display device.

In the context of this disclosure, a non-transitory computer-readable medium stores programs for use by or in connection with an instruction execution system, apparatus, or device. More specific examples of a computer-readable medium may include by way of example and without limitation: a portable computer diskette, a random access memory (RAM), a read-only memory (ROM), an erasable programmable read-only memory (EPROM, EEPROM, or Flash memory), and a portable compact disc read-only memory (CDROM) (optical).

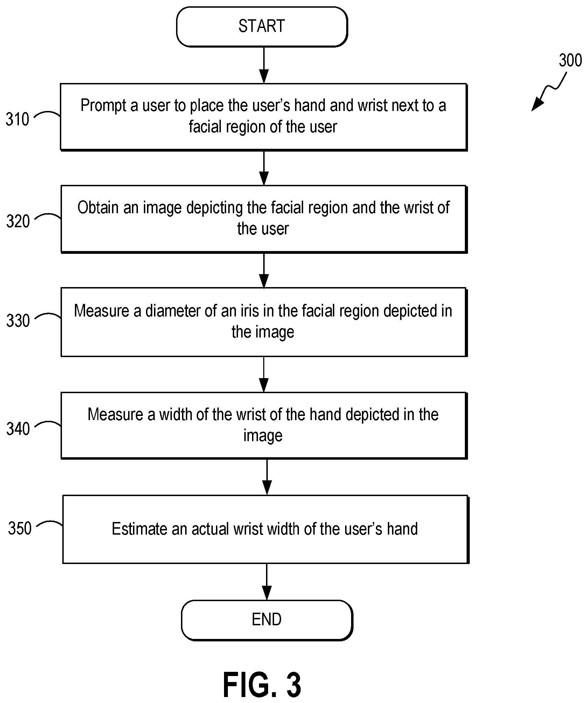

Reference is made to FIG. 3, which is a flowchart 300 in accordance with various embodiments for performing contactless wrist size estimation, where the operations are performed by the computing device 102 of FIG. 1. It is understood that the flowchart 300 of FIG. 3 provides merely an example of the different types of functional arrangements that may be employed to implement the operation of the various components of the computing device 102. As an alternative, the flowchart 300 of FIG. 3 may be viewed as depicting an example of steps of a method implemented in the computing device 102 according to one or more embodiments.

Although the flowchart 300 of FIG. 3 shows a specific order of execution, it is understood that the order of execution may differ from that which is displayed. For example, the order of execution of two or more blocks may be scrambled relative to the order shown. In addition, two or more blocks shown in succession in FIG. 3 may be executed concurrently or with partial concurrence. It is understood that all such variations are within the scope of the present disclosure.

At block 310, the computing device 102 prompts a user to place the user's hand and wrist next to a facial region of the user. At block 320, the computing device 102 obtains an image depicting the facial region and the wrist of the user. At block 330, the computing device 102 measures a diameter of an iris in the facial region depicted in the image. At block 340, the computing device 102 measures a width of the wrist of the hand depicted in the image.

At block 350, the computing device 102 estimates an actual wrist width of the user's hand based on an actual iris diameter, the measured diameter of the iris in the facial region depicted in the image, and the measured width of the wrist of the hand depicted in the image. For some embodiments, the computing device 102 estimates an actual wrist circumference based on the estimated actual wrist width and performs virtual application of an accessory on the hand based on the estimated actual wrist circumference and/or the estimated actual wrist width of the hand depicted in the image. For some embodiments, the actual wrist circumference is estimated based on the estimated actual wrist width according to the following equation:

$$estimated\ actual\ wrist\ circumference\ =\ estimated\ wrist\ width\ x\ R,$$

where R is a statistically derived ratio between an actual wrist width value and the measured wrist width.

For some embodiments, the actual wrist width of the hand is estimated based on the actual iris diameter, the measured iris diameter, and the measured wrist width, wherein the actual wrist width is estimated according to the following equation:

$$estimated\ actual\ wrist\ width\ =\ actual\ iris\ diameter\ x\ \frac{measured\ wrist\ width}{measured\ iris\ diameter},$$

where the actual iris diameter is equal to 11.7, and the estimated actual wrist width is measured in millimeters.

For some embodiments, the computing device 102 determines a wrist rotation angle ($\theta$) of the user's hand, where the actual wrist width of the hand is estimated based on the actual iris diameter, the measured iris diameter, the measured wrist width, and the wrist rotation angle. The actual wrist width is estimated according to the following equation:

$$estimated\ actual\ wrist\ width\ =\ actual\ iris\ diameter\ x\ \frac{f(measured\ wrist\ width, \theta)}{measured\ iris\ diameter},$$

where the actual iris diameter is equal to 11.7, f() is a function calculating a compensated measured wrist width based on the wrist rotation angle ($\theta$), and the estimated actual wrist width is measured in millimeters. Thereafter, the process in FIG. 3 ends.

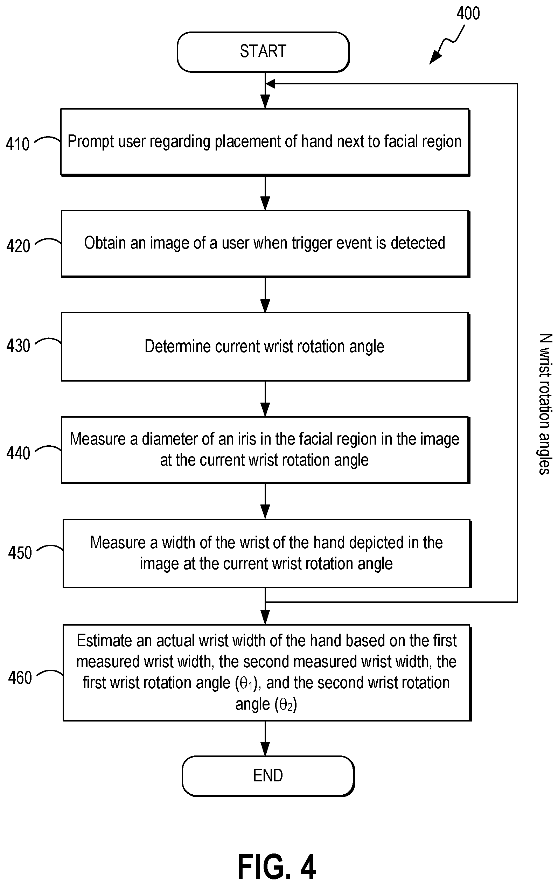

Reference is made to FIG. 4, which is a flowchart 400 in accordance with an alternative embodiment for performing contactless wrist size estimation, where the operations are performed by the computing device 102 of FIG. 1. It is understood that the flowchart 400 of FIG. 4 provides merely an example of the different types of functional arrangements that may be employed to implement the operation of the various components of the computing device 102. As an alternative, the flowchart 400 of FIG. 4 may be viewed as depicting an example of steps of a method implemented in the computing device 102 according to one or more embodiments.

Although the flowchart 400 of FIG. 4 shows a specific order of execution, it is understood that the order of execution may differ from that which is displayed. For example, the order of execution of two or more blocks may be scrambled relative to the order shown. In addition, two or more blocks shown in succession in FIG. 4 may be executed concurrently or with partial concurrence. It is understood that all such variations are within the scope of the present disclosure.

In accordance with alternative embodiments, multiple images of the user's facial region and hand are obtained, where the user's hand are held at different wrist rotation angles ($\theta_1$, $\theta_2$, *etc.*) with respect to the camera of the computing device 102 capturing the images. Each image captured by the computing device 102 corresponds to a different wrist rotation angle. In general, the wrist rotation angle ($\theta_1$) should be approximately zero ($\theta_1 = 0°$) where a value of zero for the wrist rotation angle corresponds to the user's palm either facing towards the user or the user's palm directly facing the camera. This helps to ensure that a more accurate estimated wrist size of the user is obtained. However, if the user's wrist is rotated (e.g., quarter turn), then this wrist rotation angle is taken in account, and the actual wrist circumference is determined based on the different wrist rotation angles, as described in the operations below.

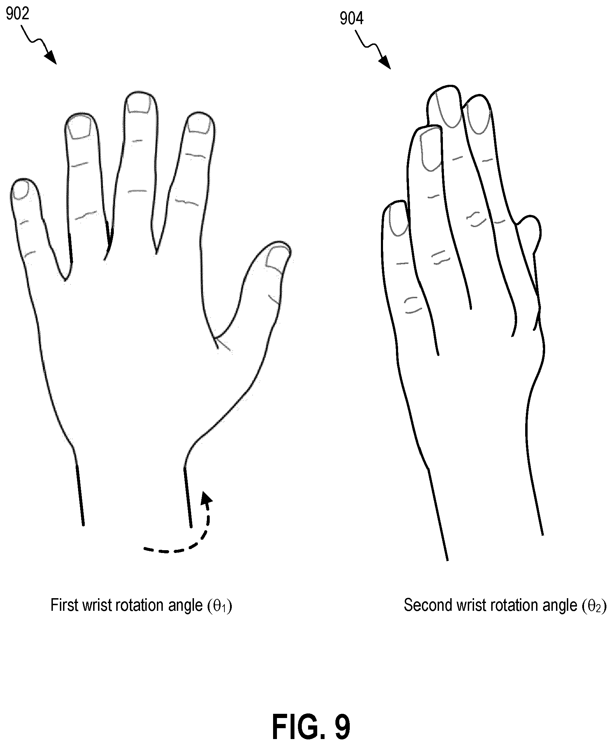

At block 410, the computing device 102 prompts a user regarding placement of the user's hand for purposes of estimating the user's wrist size. For example, the computing device 102 may prompt the user to place the user's hand next to a facial region of the user with the hand being held such that a palm of the hand faces towards the user. This corresponds to a wrist rotation angle ($\theta_1$) of zero degrees. To illustrate, reference is made to FIG. 9, which illustrates the user's hand being held at two different wrist rotation angles 902, 904. In the first wrist rotation angle ($\theta_1$) 902, the palm of the user's hand is facing the user. This corresponds to a wrist rotation angle ($\theta_1$) 902 of zero degrees.

Referring back to FIG. 4, at block 420, the computing device 102 obtains an image of a user. In particular, the computing device 102 obtains an image depicting the facial region and a wrist region of the user. At block 430, the computing device 102 determines the current wrist rotation angle ($\theta_1$) of the hand next to the user's face. At block 440, the computing device 102 measures a diameter of an iris in the facial region in the image at the current wrist rotation angle. At block 450, the computing device 102 measures a width of the wrist on the hand depicted in the image based on the wrist rotation angle ($\theta_1$) of the hand next to the user's face.

Blocks 410 to 450 may be repeated at a different wrist rotation angle ($\theta_2$). Returning to block 410, the computing device 102 prompts a user regarding placement of the user's hand for purposes of estimating the user's wrist size. In particular, the computing device 102 prompts the user to place the user's hand next to a facial region of the user with the hand being held at a different wrist rotation angle ($\theta_2$). For example, this may comprise the computing device 102 prompting the user to rotate the user's hand such that the palm of the user's hand is facing sideways relative to the camera. This corresponds to a wrist rotation angle ($\theta_2$) of approximately 90 degrees. To illustrate, reference is made to FIG. 9, which shows the user's hand being held at a second wrist rotation angle ($\theta_2$) 904 where the user's hand is slightly rotated relative to the first wrist rotation angle ($\theta_1$) 902. Referring back to FIG. 4, blocks 420 to block 450 are then performed again at the second wrist rotation angle ($\theta_2$). The number of iterations is not limited to two iterations as the operations described above may be performed at additional wrist rotation angles. For some embodiments, the operations are performed whenever the user's hand rotates.

At block 460, the computing device 102 estimates an actual wrist width of the hand based on the first measured wrist width, the second measured wrist width, the first wrist rotation angle ($\theta_1$), and the second wrist rotation angle ($\theta_2$). For some embodiments, the computing device 102 estimates the wrist width of the hand according to the following equation:

$$estimated\ actual\ wrist\ width = actual\ iris\ diameter\ x\ \frac{f_p(a,b)}{measured\ iris\ diameter},$$

wherein the actual iris diameter is equal to 11.7, $f_p()$ is a function calculating an elliptical parameter based on parameters a, b. The parameters (a) and (b) are derived according to the following equation:

$$w_1 = 2\sqrt{a^2 cos^2\theta_1 + b^2 sin^2\theta_1},$$

$$w_2 = 2\sqrt{a^2 cos^2\theta_2 + b^2 sin^2\theta_2},$$

The parameter $w_1$ is the first wrist width of the hand at the first wrist rotation angle ($\theta_1$) in the first image, and $w_2$ is the second wrist width of the hand at the second wrist rotation angle ($\theta_2$) in the second image. The estimated actual wrist width is measured in millimeters. Thereafter, the process in FIG. 4 ends.

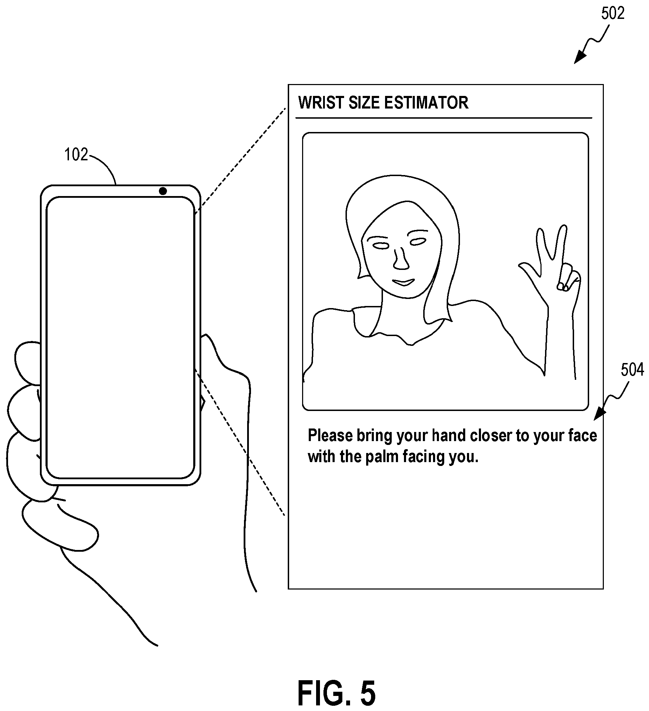

To illustrate further various aspects of the present invention, reference is made to the following figures. FIG. 5 illustrates an example user interface 502 provided on a display of the computing device 102 whereby an image of the user is displayed. As described above, the import module 106 (FIG. 1) executing in the computing device 102 can be configured to cause a camera (e.g., front-facing camera) of the computing device 102 to capture an image or a video of a user of the computing device 102. In the example user interface 502, the image depicts the user holding up the user's hand. As described above, the computing device 102 may be configured to guide the user regarding optimal placement of the user's hand for purposes of estimating the wrist size of the user.

In the user interface 502, a prompt 504 is displayed to the user instructing the user on where to place the user's hand relative to the facial region of the user. Preferably, the wrist rotation angle ($\theta$) should be approximately zero ($\theta = 0°$) where a value of zero for the wrist rotation angle corresponds to the user's palm either facing towards the user or the user's palm directly facing the camera. This helps to ensure that a more accurate estimated wrist size of the user is obtained. If the user's wrist is partially rotated (e.g., quarter turn), then the wrist rotation angle ($\theta$) is taken into account when estimating the wrist width, as described above.

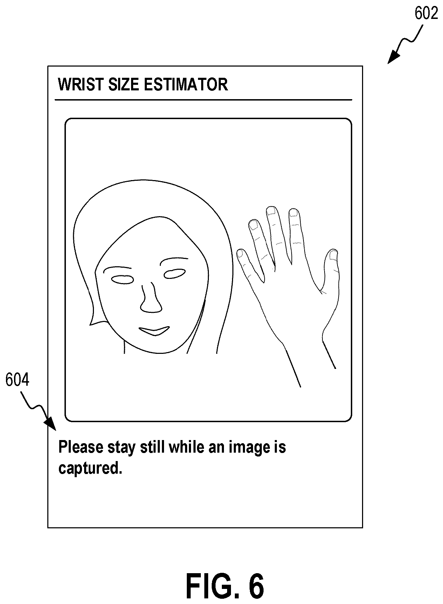

FIG. 6 illustrates an example user interface 602 for guiding the user regarding hand placement according to various embodiments of the present disclosure. When the proximity sensor 107 (FIG. 1) executing in the computing device 102 (FIG. 1) senses that the user's hand is within a threshold distance of the user's facial region, the user interface 602 displays a prompt 604 instructing the user to remain still while the import module 106 (FIG. 1) causes a front-facing camera of the computing device 102 to capture an image of the user. The facial region tracker 108 (FIG. 1) will then analyze the facial region depicted in the captured image, while the hand region tracker 110 (FIG. 1) will analyze the hand region depicted in the captured image in order to estimate the wrist size of the user.

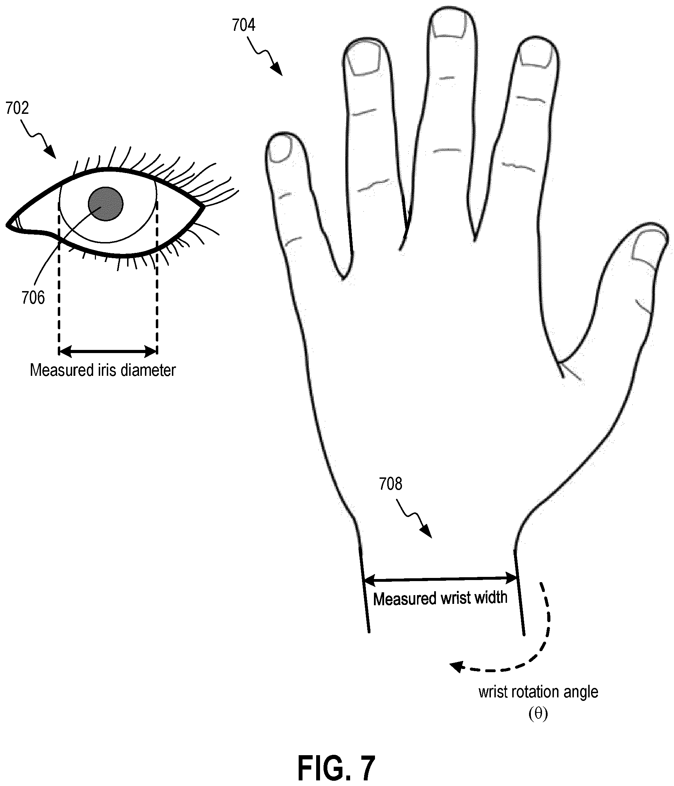

FIG. 7 illustrates the computing device 102 of FIG. 1 estimating the wrist size of the user based on the measured iris diameter and the measured wrist width of the user. The facial region tracker 108 (FIG. 1) identifies an eye 702 of the user closest to the user's hand 704 and identifies the iris 706 of the user's eye 702. The facial region tracker 108 then measures the width of the iris 706 in pixels. The hand region tracker 110 (FIG. 1) analyzes the orientation of the user's hand 704 and identifies the wrist region on the hand 704. The hand region tracker 110 then measures the width of the wrist 708 based on the orientation of the user's hand 704.

The wrist size estimator 112 (FIG. 1) executing in the computing device 102 then estimates the wrist size based on the actual iris diameter, the measured iris diameter, and the measured wrist width 708, wherein the actual wrist width is estimated according to the following equation:

$$estimated\ actual\ wrist\ width = actual\ iris\ diameter\ x\ \frac{measured\ wrist\ width}{measured\ iris\ diameter},$$

where the actual iris diameter is equal to 11.7, and where the estimated actual wrist size is measured in millimeters. A value of 11.7 mm is used for the actual iris diameter for various embodiments because the average white-to-white horizontal diameter of the eye is approximately 11.7 mm $\pm$ 0.5 mm. The computing device 102 utilizes the 11.7 mm as a reference value when estimating the wrist size of the user.

For some embodiments, the hand region tracker 110 determines a wrist rotation angle ($\theta$) to obtain a more accurate estimation of the wrist size by taking into the account how the user's hand is oriented. Preferably, the wrist rotation angle ($\theta$) should be approximately zero ($\theta= 0°$). A value of zero for the wrist rotation angle corresponds to the user's palm either facing towards the user or the user's palm directly facing the camera. This helps to ensure that a more accurate estimated wrist size of the user is obtained. If the user's wrist is partially rotated (e.g., quarter turn), then the wrist rotation angle ($\theta$) is taken into account when estimating the wrist width. Specifically, the wrist size estimator 112 estimates the wrist width of the hand based on the actual iris diameter, the measured iris diameter, the measured wrist width, and the wrist rotation angle, where the actual wrist width is estimated according to the following equation:

$$estimated\ actual\ wrist\ width = actual\ iris\ diameter\ x\ \frac{f(\theta, measured\ wrist\ width)}{measured\ iris\ diameter},$$

where the actual iris diameter is equal to 11.7, f() is a function calculating a compensated measured wrist width based on the wrist rotation angle ($\theta$), and where the estimated actual wrist width is measured in millimeters.

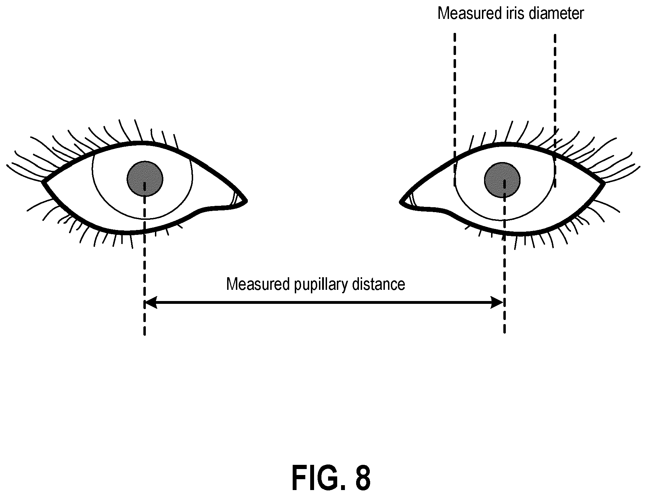

FIG. 8 illustrates the computing device 102 of FIG. 1 estimating the wrist size of the user based on the measured iris diameter and the measured pupillary distance of the user. In some instances, the user may know the actual pupillary distance of the user based on, for example, a glasses prescription as this measurement is used to determine where the user looks through the lens of the user's glasses. The pupillary distance is the distance between the centers of the user's pupils. For some embodiments, the user can enter the actual pupillary distance, and the wrist size estimator 112 (FIG. 1) estimates the wrist size based on the actual pupillary distance, the measured wrist width, and the measured pupillary distance, where the actual wrist width is estimated according to the following equation:

$$estimated\ actual\ wrist\ width$$
$$= actual\ pupillary\ distance\ x\ \frac{measured\ wrist\ width}{measured\ pupillary\ distance},$$

where the estimated actual wrist size is measured in millimeters.

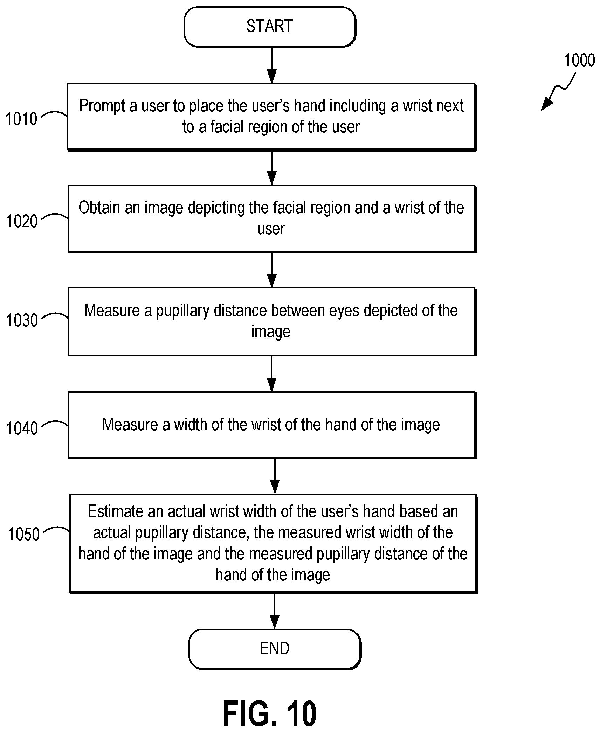

Reference is made to FIG. 10, which is a flowchart 1000 in accordance with another alternative embodiment for performing contactless wrist size estimation, where the operations are performed by the computing device 102 of FIG. 1. It is understood that the flowchart 1000 of FIG. 10 provides merely an example of the different types of functional arrangements that may be employed to implement the operation of the various components of the computing device 102. As an alternative, the flowchart 1000 of FIG. 10 may be viewed as depicting an example of steps of a method implemented in the computing device 102 according to one or more embodiments.

Although the flowchart 1000 of FIG. 10 shows a specific order of execution, it is understood that the order of execution may differ from that which is displayed. For example, the order of execution of two or more blocks may be scrambled relative to the order shown. In addition, two or more blocks shown in succession in FIG. 10 may be executed concurrently or with partial concurrence. It is understood that all such variations are within the scope of the present disclosure.

At block 1010, the computing device prompts a user to place the user's hand including a wrist next to a facial region of the user. At block 1020, the computing device obtains an image depicting the facial region and the wrist of the user. At block 1030, the computing device measures a pupillary distance between eyes depicted of the image. At block 1040, the computing device measures a width of the wrist of the hand depicted in the image. At block 1050, the computing device estimates an actual wrist width of the user's hand based on an actual pupillary distance, the measured wrist width of the hand depicted in the image, and the measured pupillary distance of the hand depicted in the image.

For some embodiments, the computing device estimates the actual wrist width based on the actual pupillary distance, the measured wrist width, and the measured pupillary distance, wherein the actual wrist width is estimated according to the following equation:

$$estimated\ actual\ wrist\ width = actual\ pupillary\ distance\ x\ \frac{measured\ wrist\ width}{measured\ pupillary\ distance},$$

where the actual pupillary distance is specified by the user.

For some embodiments, the computing device estimates an actual wrist circumference based on the estimated actual wrist width and performs virtual application of an accessory on the hand based on at least one of: the estimated actual wrist circumference, or the estimated actual wrist width of the hand depicted in the image. Thereafter, the process in FIG. 10 ends.

**Claims**

1. A method implemented in a computing device (102), comprising:

   prompting (310) a user to place the user's hand (704) and wrist (708) in close proximity a facial region of the user;
   obtaining (320) an image depicting the facial region and the wrist of the user;
   measuring (330) a diameter of an iris (706) in the facial region depicted in the image to obtain a measured diameter of the iris in the facial region depicted in the image;
   measuring (340) a width of the wrist of the hand depicted in the image to obtain a measured width of the wrist of the hand depicted in the image; and
   estimating (350) an actual wrist width of the user's hand based on an actual iris diameter, the measured diameter of the iris in the facial region depicted in the image, and the measured width of the wrist of the hand depicted in the image to obtain an estimated actual wrist width of the user's hand.

2. The method of claim 1, wherein the estimating (350) the actual wrist width of the user's hand is performed according to the following equation:

$$estimated\ actual\ wrist\ width$$
$$= actual\ iris\ diameter\ x\ \frac{measured\ wrist\ width}{measured\ iris\ diameter},$$

wherein the actual iris diameter is equal to 11.7, and wherein the estimated actual wrist width of the user's hand is measured in millimeters.

3. The method of claim 1, further comprising:

   estimating an actual wrist circumference of the user's hand based on the estimated actual wrist width of the user's hand to obtain an estimated actual wrist circumference; and
   performing a virtual application of an accessory on the hand based on at least one of: an estimated actual wrist circumference and the estimated actual wrist width of the user's hand.

4. The method of claim 3, wherein the estimated actual wrist circumference is estimated based on the estimated actual wrist width of the user's hand according to the following equation:

$$estimated\ actual\ wrist\ circumference\ =\ estimated\ actual\ wrist\ width\ x\ R,$$

wherein R is a statistically derived ratio between an actual wrist width value and the actual wrist circumference.

5. A method implemented in a computing device (102), comprising:

prompting (1010) a user to place the user's hand (704) and wrist (708) in close proximity a facial region of the user;
obtaining (1020) an image depicting the facial region and the wrist of the user;
measuring (1030) a pupillary distance between eyes (702) depicted in the image to obtain a measured pupillary distance between the eyes (702) depicted in the image;
measuring (1040) a width of the wrist of the hand depicted in the image to obtain a measured wrist width of the hand depicted in the image; and
estimating (1050) an actual wrist width of the user's hand based on an actual pupillary distance, the measured wrist width of the hand depicted in the image, and the measured pupillary distance between the eyes (702) depicted in the image to obtain an estimated actual wrist width of the user's hand.

6. The method of claim 5, further comprising:

estimating an actual wrist circumference of the user's hand based on the estimated actual wrist width of the user's hand to obtain an estimated actual wrist circumference; and
performing a virtual application of an accessory on the hand based on at least one of: the estimated actual wrist circumference, or the estimated actual wrist width of the user's hand.

7. The method of claim 5,
wherein the estimated actual wrist width of the user's hand is estimated according to the following equation:

$$estimated\ actual\ wrist\ width$$
$$=\ actual\ pupillary\ distance\ x\ \frac{measured\ wrist\ width}{measured\ pupillary\ distance},$$

wherein the actual pupillary distance is specified by the user.

8. A system, comprising:

a memory (214) storing instructions;
a processor (202) coupled to the memory (214) and configured by the instructions to at least:

prompt (310) a user to place the user's hand (704) including a wrist (708) of the user in close proximity a facial region of the user;
obtain (320) an image depicting the facial region and the wrist of the user;
measure (330) a diameter of an iris (706) in the facial region depicted in the image to obtain a measured diameter of the iris in the facial region depicted in the image ;
measure (340) a width of the wrist of the hand depicted in the image to obtain a measured width of the wrist of the hand depicted in the image; and
estimate (350) an actual wrist width of the user's hand based on an actual iris diameter, the measured diameter of the iris in the facial region depicted in the image, and the measured width of the wrist of the hand depicted in the image to obtain an estimated actual wrist width of the user's hand.

9. The system of claim 8, wherein the processor (202) is configured to estimate an actual wrist circumference of the user's hand based on the estimated actual wrist width of the user's hand to obtain an estimated actual wrist circumference; and to perform a virtual application of an accessory on the hand based on at least one of: the estimated actual wrist circumference, or the estimated actual wrist width of the user's hand.

10. The system of claim 9, wherein the processor (202) is configured to estimate the estimated actual wrist circumference based on the estimated actual wrist width according to the following equation:

$$estimated\ actual\ wrist\ circumference = estimated\ actual\ wrist\ width\ x\ R,$$

wherein R is a statistically derived ratio between an actual wrist width value and the actual wrist circumference.

**11.** The system of claim 8, wherein the processor (202) is configured to estimate the wrist width of the user's hand according to the following equation:

$$estimated\ actual\ wrist\ width$$
$$= actual\ iris\ diameter\ x\ \frac{measured\ wrist\ width}{measured\ iris\ diameter},$$

wherein the actual iris diameter is equal to 11.7, and wherein the estimated actual wrist width of the user's hand is measured in millimeters.

**12.** A non-transitory computer-readable storage medium storing instructions to be implemented by a computing device (102) having a processor (202), wherein the instructions, when executed by the processor (202), cause the computing device (102) to at least:

prompt (310) a user to place the user's hand (704) including a wrist (708) in close proximity a facial region of the user;
obtain (320) an image depicting the facial region and the wrist of the user;
measure (330) a diameter of an iris (706) in the facial region depicted in the image to obtain a measured diameter of the iris in the facial region depicted in the image;
measure (340) a width of the wrist of the hand depicted in the image to obtain a measured width of the wrist of the hand depicted in the image; and
estimate (350) an actual wrist width of the user's hand based on an actual iris diameter, the measured diameter of the iris in the facial region depicted in the image and the measured width of the wrist of the hand depicted in the image to obtain an estimated actual wrist width of the user's hand.

**13.** The non-transitory computer-readable storage medium of claim 12, wherein the processor (202) is further configured to:

estimating an actual wrist circumference of the user's hand based on the estimated actual wrist width of the user's hand to obtain an estimated actual wrist circumference of the user's hand; and
performing a virtual application of an accessory on the hand based on at least one of: the estimated actual wrist circumference of the user's hand and the estimated actual wrist width of the user's hand.

**14.** The non-transitory computer-readable storage medium of claim 13,
wherein the processor (202) is configured to estimate the estimated actual wrist circumference based on the estimated actual wrist width of the user's hand according to the following equation:

$$estimated\ actual\ wrist\ circumference = estimated\ actual\ wrist\ width\ x\ R,$$

wherein R is a statistically derived ratio between an actual wrist width value and the actual wrist circumference.

**15.** A method implemented in a computing device (102), comprising:

prompting (410) a user to place the user's hand (704) in close proximity a facial region of the user with the hand being held in a first roll orientation and then with the hand being held in a second roll orientation;
obtaining (420) a first image at the first roll orientation and a second image at the second roll orientation, the first image and the second image each depicting the facial region and a wrist (708) of the user;
determining (430) a first wrist rotation angle ($\theta_1$) at the first roll orientation in the first image and a second wrist rotation angle ($\theta_2$) at the second roll orientation in the second image;

measuring (440) a first diameter of an iris (706) in the facial region at the first roll orientation in the first image and a second diameter of the iris in the facial region at the second roll orientation in the second image;

measuring (450) a first width of the wrist of the hand at the first roll orientation in the first image to obtain a first measured wrist width and a second width of the wrist of the hand at the second roll orientation in the second image to obtain a second measured wrist width; and

estimating (460) an actual wrist width of the user's hand based on the first measured wrist width, the second measured wrist width, the first wrist rotation angle ($\theta_1$), and the second wrist rotation angle ($\theta_2$) to obtain an estimated actual wrist width of the user's hand.

16. The method of claim 15, further comprising:

estimating an actual wrist circumference of the user's hand based on the estimated actual wrist width of the user's hand to obtain an estimated actual wrist circumference; and

performing a virtual application of an accessory on the hand based on at least one of: the estimated actual wrist circumference and the estimated actual wrist width of the user's hand.

17. A system, comprising:

a memory (214) storing instructions;

a processor (202) coupled to the memory (214) and configured by the instructions to at least:

prompt (1010) a user to place the user's hand (704) and wrist (708) in close proximity a facial region of the user;

obtain (1020) an image depicting the facial region and the wrist of the user;

measure (1030) a pupillary distance between eyes (702) depicted in the image to obtain a measured pupillary distance between the eyes (702) depicted in the image;

measure (1040) a width of the wrist of the hand depicted in the image to obtain a measured wrist width of the hand depicted in the image; and

estimate (1050) an actual wrist width of the user's hand based on an actual pupillary distance, the measured wrist width of the hand depicted in the image, and the measured pupillary distance between the eyes (702) depicted in the image.

18. A system, comprising:

a memory (214) storing instructions;

a processor (202) coupled to the memory (214) and configured by the instructions to at least:

prompt (410) a user to place the user's hand (704) in close proximity a facial region of the user with the hand being held in a first roll orientation and then with the hand being held in a second roll orientation;

obtain (420) a first image at the first roll orientation and a second image at the second roll orientation, the first image and the second image each depicting the facial region and a wrist (708) of the user;

determine (430) a first wrist rotation angle ($\theta_1$) at the first roll orientation in the first image and a second wrist rotation angle ($\theta_2$) at the second roll orientation in the second image;

measure (440) a first diameter of an iris (706) in the facial region at the first roll orientation in the first image and a second diameter of the iris in the facial region at the second roll orientation in the second image;

measure (450) a first width of the wrist of the hand at the first roll orientation in the first image to obtain a first measured wrist width and a second width of the wrist of the hand at the second roll orientation in the second image to obtain a second measured wrist width; and

estimate (460) an actual wrist width of the user's hand based on the first measured wrist width, the second measured wrist width, the first wrist rotation angle ($\theta_1$), and the second wrist rotation angle ($\theta_2$).

19. A non-transitory computer-readable storage medium storing instructions to be implemented by a computing device (102) having a processor (202), wherein the instructions, when executed by the processor (202), cause the computing device (102) to at least:

prompting (1010) a user to place the user's hand (704) and wrist (708) in close proximity a facial region of the user;

obtaining (1020) an image depicting the facial region and the wrist of the user;

measuring (1030) a pupillary distance between eyes (702) depicted in the image to obtain a measured pupillary distance between the eyes depicted in the image;

measuring (1040) a width of the wrist of the hand depicted in the image to obtain a measured wrist width of the hand depicted in the image; and

estimating (1050) an actual wrist width of the user's hand based on an actual pupillary distance, the measured wrist width of the hand depicted in the image, and the measured pupillary distance between the eyes (702) depicted in the image.

20. A non-transitory computer-readable storage medium storing instructions to be implemented by a computing device (102) having a processor (202), wherein the instructions, when executed by the processor (202), cause the computing device (102) to at least:

prompting (410) a user to place the user's hand (704) in close proximity a facial region of the user with the hand being held in a first roll orientation and then with the hand being held in a second roll orientation;

obtaining (420) a first image at the first roll orientation and a second image at the second roll orientation, the first image and the second image each depicting the facial region and a wrist (708) of the user;

determining (430) a first wrist rotation angle ($\theta_1$) at the first roll orientation in the first image and a second wrist rotation angle ($\theta_2$) at the second roll orientation in the second image;

measuring (440) a first diameter of an iris (706) in the facial region at the first roll orientation in the first image and a second diameter of the iris in the facial region at the second roll orientation in the second image;

measuring (450) a first width of the wrist of the hand at the first roll orientation in the first image to obtain a first measured wrist width and a second width of the wrist of the hand at the second roll orientation in the second image to obtain a second measured wrist width; and

estimating (460) an actual wrist width of the user's hand based on the first measured wrist width, the second measured wrist width, the first wrist rotation angle ($\theta_1$), and the second wrist rotation angle ($\theta_2$).

**Patentansprüche**

1. Ein Verfahren, das in einer Computervorrichtung (102) implementiert ist und folgende Schritte aufweist:

Auffordern (310) eines Benutzers, die Hand (704) und das Handgelenk (708) des Benutzers in unmittelbarer Nähe einer Gesichtsregion des Benutzers zu positionieren;

Erhalten (320) eines Bildes, das die Gesichtsregion und das Handgelenk des Benutzers darstellt;

Messen (330) eines Durchmessers einer Iris (706) in der Gesichtsregion, die in dem Bild dargestellt ist, um einen gemessenen Durchmesser der Iris in der Gesichtsregion, die in dem Bild dargestellt ist, zu erhalten;

Messen (340) einer Breite des Handgelenks der Hand, die in dem Bild dargestellt ist, um eine gemessene Breite des Handgelenks der Hand, die in dem Bild dargestellt ist, zu erhalten; und

Schätzen (350) einer tatsächlichen Handgelenksbreite der Hand des Benutzers basierend auf einem tatsächlichen Irisdurchmesser, dem gemessenen Durchmesser der Iris in der Gesichtsregion, die in dem Bild dargestellt ist, und der gemessenen Breite des Handgelenks der Hand, die in dem Bild dargestellt ist, um eine geschätzte tatsächliche Handgelenksbreite der Hand des Benutzers zu erhalten.

2. Das Verfahren gemäß Anspruch 1, wobei das Schätzen (350) der tatsächlichen Handgelenksbreite der Hand des Benutzers gemäß der folgenden Gleichung durchgeführt wird:

$$\textit{geschätzte tatsächliche Handgelenksbreite} = \textit{tatsächlicher Irisdurchmesser} \; x \; \frac{\textit{gemessene Handgelenksbreite}}{\textit{gemessener Irisdurchmesser}},$$

wobei der tatsächliche Irisdurchmesser gleich 11,7 ist und wobei die geschätzte tatsächliche Handgelenksbreite der Hand des Benutzers in Millimetern gemessen wird.

3. Das Verfahren gemäß Anspruch 1, das ferner folgende Schritte aufweist:

Schätzen eines tatsächlichen Handgelenksumfangs der Hand des Benutzers basierend auf der geschätzten tatsächlichen Handgelenksbreite der Hand des Benutzers, um einen geschätzten tatsächlichen Handgelenksumfang zu erhalten; und

Durchführen eines virtuellen Anbringens eines Zubehörteils an der Hand basierend auf zumindest einem aus einem geschätzten tatsächlichen Handgelenksumfang und der geschätzten tatsächlichen Handgelenksbreite der Hand des Benutzers.

4.  Das Verfahren gemäß Anspruch 3, wobei der geschätzte tatsächliche Handgelenksumfang basierend auf der geschätzten tatsächlichen Handgelenksbreite der Hand des Benutzers gemäß der folgenden Gleichung geschätzt wird:

$$gesch\ddot{a}tzter\ tats\ddot{a}chlicher\ Handgelenksumfang$$
$$= gesch\ddot{a}tzte\ tats\ddot{a}chliche\ Handgelenksbreite\ x\ R,$$

wobei R ein statistisch abgeleitetes Verhältnis aus einem tatsächlichen Handgelenksbreitenwert und dem tatsächlichen Handgelenksumfang ist.

5.  Ein Verfahren, das in einer Computervorrichtung (102) implementiert ist und folgende Schritte aufweist:

Auffordern (1010) eines Benutzers, die Hand (704) und das Handgelenk (708) des Benutzers in unmittelbarer Nähe einer Gesichtsregion des Benutzers zu positionieren;
Erhalten (1020) eines Bildes, das die Gesichtsregion und das Handgelenk des Benutzers darstellt;
Messen (1030) eines Pupillenabstands zwischen Augen (702), die in dem Bild dargestellt sind, um einen gemessenen Pupillenabstand zwischen den Augen (702), die in dem Bild dargestellt sind, zu erhalten;
Messen (1040) einer Breite des Handgelenks der Hand, die in dem Bild dargestellt ist, um eine gemessene Handgelenksbreite der Hand, die in dem Bild dargestellt ist, zu erhalten; und
Schätzen (1050) einer tatsächlichen Handgelenksbreite der Hand des Benutzers basierend auf einem tatsächlichen Pupillenabstand, der gemessenen Handgelenksbreite der Hand, die in dem Bild dargestellt ist, und dem gemessenen Pupillenabstand zwischen den Augen (702), die in dem Bild dargestellt sind, um eine geschätzte tatsächliche Handgelenksbreite der Hand des Benutzers zu erhalten.

6.  Das Verfahren gemäß Anspruch 5, das ferner folgende Schritte aufweist:

Schätzen eines tatsächlichen Handgelenksumfangs der Hand des Benutzers basierend auf der geschätzten tatsächlichen Handgelenksbreite der Hand des Benutzers, um einen geschätzten tatsächlichen Handgelenksumfang zu erhalten; und
Durchführen eines virtuellen Anbringens eines Zubehörteils an der Hand basierend auf zumindest einem aus dem geschätzten tatsächlichen Handgelenksumfang und der geschätzten tatsächlichen Handgelenksbreite der Hand des Benutzers.

7.  Das Verfahren gemäß Anspruch 5,

wobei die geschätzte tatsächliche Handgelenksbreite der Hand des Benutzers gemäß der folgenden Gleichung geschätzt wird:

$$gesch\ddot{a}tzte\ tats\ddot{a}chliche\ Handgelenksbreite$$
$$= tats\ddot{a}chlicher\ Pupillenabstand\ x\ \frac{gemessene\ Handgelenksbreite}{gemessener\ Pupillenabstand},$$

wobei der tatsächliche Pupillenabstand durch den Benutzer spezifiziert wird.

8.  Ein System, das folgende Merkmale aufweist:

einen Speicher (214), in dem Anweisungen gespeichert sind,
einen Prozessor (202), der mit dem Speicher (214) gekoppelt ist und durch die Anweisungen zumindest zu Folgendem konfiguriert ist.

Auffordern (310) eines Benutzers, die Hand (704) des Benutzers, einschließlich eines Handgelenks (708) des Benutzers, in unmittelbarer Nähe einer Gesichtsregion des Benutzers zu positionieren;

Erhalten (320) eines Bildes, das die Gesichtsregion und das Handgelenk des Benutzers darstellt;
Messen (330) eines Durchmessers einer Iris (706) in der Gesichtsregion, die in dem Bild dargestellt ist, um einen gemessenen Durchmesser der Iris in der Gesichtsregion, die in dem Bild dargestellt ist, zu erhalten;
Messen (340) einer Breite des Handgelenks der Hand, die in dem Bild dargestellt ist, um eine gemessene Breite des Handgelenks der Hand, die in dem Bild dargestellt ist, zu erhalten; und
Schätzen (350) einer tatsächlichen Handgelenksbreite der Hand des Benutzers basierend auf einem tatsächlichen Irisdurchmesser, dem gemessenen Durchmesser der Iris in der Gesichtsregion, die in dem Bild dargestellt ist, und der gemessenen Breite des Handgelenks der Hand, die in dem Bild dargestellt ist, um eine geschätzte tatsächliche Handgelenksbreite der Hand des Benutzers zu erhalten.

9. Das System gemäß Anspruch 8, wobei der Prozessor (202) dazu konfiguriert ist, einen tatsächlichen Handgelenksumfang der Hand des Benutzers basierend auf der geschätzten tatsächlichen Handgelenksbreite der Hand des Benutzers, um einen geschätzten tatsächlichen Handgelenksumfang zu erhalten, und ein virtuelles Anbringen eines Zubehörteils an der Hand basierend auf zumindest einem aus dem geschätzten tatsächlichen Handgelenksumfang und der geschätzten tatsächlichen Handgelenksbreite der Hand des Benutzers durchzuführen.

10. Das System gemäß Anspruch 9, wobei der Prozessor (202) dazu konfiguriert ist, den geschätzten tatsächlichen Handgelenksumfang basierend auf der geschätzten tatsächlichen Handgelenksbreite gemäß der folgenden Gleichung zu schätzen:

$$gesch\ddot{a}tzter\ tats\ddot{a}chlicher\ Handgelenksumfang$$
$$= gesch\ddot{a}tzte\ tats\ddot{a}chliche\ Handgelenksbreite\ x\ R,$$

wobei R ein statistisch abgeleitetes Verhältnis aus einem tatsächlichen Handgelenksbreitenwert und dem tatsächlichen Handgelenksumfang ist.

11. Das System gemäß Anspruch 8, wobei der Prozessor (202) dazu konfiguriert ist, die Handgelenksbreite der Hand des Benutzers gemäß der folgenden Gleichung zu schätzen:

$$gesch\ddot{a}tzte\ tats\ddot{a}chliche\ Handgelenksbreite$$
$$= tats\ddot{a}chlicher\ Irisdurchmesser\ x\ \frac{gemessene\ Handgelenksbreite}{gemessener\ Irisdurchmesser},$$

wobei der tatsächliche Irisdurchmesser gleich 11,7 ist und wobei die geschätzte tatsächliche Handgelenksbreite der Hand des Benutzers in Millimetern gemessen wird.

12. Ein nicht flüchtiges, computerlesbares Speichermedium, auf dem Anweisungen gespeichert sind, die durch eine Computervorrichtung (102) mit einem Prozessor (202) zu implementieren sind, wobei die Anweisungen bei Ausführung durch den Prozessor (202) bewirken, dass die Computervorrichtung (102) zumindest folgende Schritte durchführt:

Auffordern (310) eines Benutzers, die Hand (704) des Benutzers, einschließlich eines Handgelenks (708), in unmittelbarer Nähe einer Gesichtsregion des Benutzers zu positionieren;
Erhalten (320) eines Bildes, das die Gesichtsregion und das Handgelenk des Benutzers darstellt;
Messen (330) eines Durchmessers einer Iris (706) in der Gesichtsregion, die in dem Bild dargestellt ist, um einen gemessenen Durchmesser der Iris in der Gesichtsregion, die in dem Bild dargestellt ist, zu erhalten;
Messen (340) einer Breite des Handgelenks der Hand, die in dem Bild dargestellt ist, um eine gemessene Breite des Handgelenks der Hand, die in dem Bild dargestellt ist, zu erhalten; und
Schätzen (350) einer tatsächlichen Handgelenksbreite der Hand des Benutzers basierend auf einem tatsächlichen Irisdurchmesser, dem gemessenen Durchmesser der Iris in der Gesichtsregion, die in dem Bild dargestellt ist, und der gemessenen Breite des Handgelenks der Hand, die in dem Bild dargestellt ist, um eine geschätzte tatsächliche Handgelenksbreite der Hand des Benutzers zu erhalten.

13. Das nicht flüchtige, computerlesbare Speichermedium gemäß Anspruch 12, wobei der Prozessor (202) zu Folgendem konfiguriert ist:

Schätzen eines tatsächlichen Handgelenksumfangs der Hand des Benutzers basierend auf der geschätzten tatsächlichen Handgelenksbreite der Hand des Benutzers, um einen geschätzten tatsächlichen Handgelenksumfang der Hand des Benutzers zu erhalten; und

Durchführen eines virtuellen Anbringens eines Zubehörteils an der Hand basierend auf zumindest einem aus dem geschätzten tatsächlichen Handgelenksumfang der Hand des Benutzers und der geschätzten tatsächlichen Handgelenksbreite der Hand des Benutzers.

**14.** Das nicht flüchtige, computerlesbare Speichermedium gemäß Anspruch 13,

wobei der Prozessor (202) dazu konfiguriert ist, den geschätzten tatsächlichen Handgelenksumfang basierend auf der geschätzten tatsächlichen Handgelenksbreite der Hand des Benutzers gemäß der folgenden Gleichung zu schätzen:

$$gesch\ddot{a}tzter\ tats\ddot{a}chlicher\ Handgelenksumfang$$

$$= gesch\ddot{a}tzte\ tats\ddot{a}chliche\ Handgelenksbreite\ x\ R,$$

wobei R ein statistisch abgeleitetes Verhältnis aus einem tatsächlichen Handgelenksbreitenwert und dem tatsächlichen Handgelenksumfang ist.

**15.** Ein Verfahren, das in einer Computervorrichtung (102) implementiert ist und folgende Schritte aufweist:

Auffordern (410) eines Benutzers, die Hand (704) des Benutzers in unmittelbarer Nähe einer Gesichtsregion des Benutzers zu positionieren, wobei die Hand in einer ersten Rollausrichtung gehalten wird und wobei die Hand anschließend in einer zweiten Rollausrichtung gehalten wird;

Erhalten (420) eines ersten Bildes bei der ersten Rollausrichtung und eines zweiten Bildes bei der zweiten Rollausrichtung, wobei das erste Bild und das zweite Bild jeweils die Gesichtsregion und ein Handgelenk (708) des Benutzers darstellen;

Bestimmen (430) eines ersten Handgelenkdrehwinkels ($\theta_1$) bei der ersten Rollausrichtung in dem ersten Bild und eines zweiten Handgelenkdrehwinkels ($\theta_2$) bei der zweiten Rollausrichtung in dem zweiten Bild;

Messen (440) eines ersten Durchmessers einer Iris (706) in der Gesichtsregion bei der ersten Rollausrichtung in dem ersten Bild und eines zweiten Durchmessers der Iris in der Gesichtsregion bei der zweiten Rollausrichtung in dem zweiten Bild;

Messen (450) einer ersten Breite des Handgelenks der Hand bei der ersten Rollausrichtung in dem ersten Bild, um eine erste gemessene Handgelenksbreite zu erhalten, und einer zweiten Breite des Handgelenks der Hand bei der zweiten Rollausrichtung in dem zweiten Bild, um eine zweite gemessene Handgelenksbreite zu erhalten; und

Schätzen (460) einer tatsächlichen Handgelenksbreite der Hand des Benutzers basierend auf der ersten gemessenen Handgelenksbreite, der zweiten gemessenen Handgelenksbreite, des ersten Handgelenkdrehwinkels ($\theta_1$) und des zweiten Handgelenkdrehwinkels ($\theta_2$), um eine geschätzte tatsächliche Handgelenksbreite der Hand des Benutzers zu erhalten.

**16.** Das Verfahren gemäß Anspruch 15, das ferner folgende Schritte aufweist:

Schätzen eines tatsächlichen Handgelenksumfangs der Hand des Benutzers basierend auf der geschätzten tatsächlichen Handgelenksbreite der Hand des Benutzers, um einen geschätzten tatsächlichen Handgelenksumfang zu erhalten; und

Durchführen eines virtuellen Anbringens eines Zubehörteils an der Hand basierend auf zumindest einem aus dem geschätzten tatsächlichen Handgelenksumfang und der geschätzten tatsächlichen Handgelenksbreite der Hand des Benutzers.

**17.** Ein System, das folgende Merkmale aufweist:

einen Speicher (214), in dem Anweisungen gespeichert sind,

einen Prozessor (202), der mit dem Speicher (214) gekoppelt ist und durch die Anweisungen zumindest zu Folgendem konfiguriert ist.

Auffordern (1010) eines Benutzers, die Hand (704) und das Handgelenk (708) des Benutzers in unmittelbarer Nähe einer Gesichtsregion des Benutzers zu positionieren;

Erhalten (1020) eines Bildes, das die Gesichtsregion und das Handgelenk des Benutzers darstellt;

Messen (1030) eines Pupillenabstands zwischen Augen (702), die in dem Bild dargestellt sind, um einen gemessenen Pupillenabstand zwischen den Augen (702), die in dem Bild dargestellt sind, zu erhalten;

Messen (1040) einer Breite des Handgelenks der Hand, die in dem Bild dargestellt ist, um eine gemessene Handgelenksbreite der Hand, die in dem Bild dargestellt ist, zu erhalten; und

Schätzen (1050) einer tatsächlichen Handgelenksbreite der Hand des Benutzers basierend auf einem tatsächlichen Pupillenabstand, der gemessenen Handgelenksbreite der Hand, die in dem Bild dargestellt ist, und dem gemessenen Pupillenabstand zwischen den Augen (702), die in dem Bild dargestellt sind.

18. Ein System, das folgende Merkmale aufweist:

einen Speicher (214), in dem Anweisungen gespeichert sind,

einen Prozessor (202), der mit dem Speicher (214) gekoppelt ist und durch die Anweisungen zumindest zu Folgendem konfiguriert ist.

Auffordern (410) eines Benutzers, die Hand (704) des Benutzers in unmittelbarer Nähe einer Gesichtsregion des Benutzers zu positionieren, wobei die Hand in einer ersten Rollausrichtung gehalten wird und wobei die Hand anschließend in einer zweiten Rollausrichtung gehalten wird;

Erhalten (420) eines ersten Bildes bei der ersten Rollausrichtung und eines zweiten Bildes bei der zweiten Rollausrichtung, wobei das erste Bild und das zweite Bild jeweils/beide die Gesichtsregion und ein Handgelenk (708) des Benutzers darstellen;

Bestimmen (430) eines ersten Handgelenkdrehwinkels ($\theta_1$) bei der ersten Rollausrichtung in dem ersten Bild und eines zweiten Handgelenkdrehwinkels ($\theta_2$) bei der zweiten Rollausrichtung in dem zweiten Bild;

Messen (440) eines ersten Durchmessers einer Iris (706) in der Gesichtsregion bei der ersten Rollausrichtung in dem ersten Bild und eines zweiten Durchmessers der Iris in der Gesichtsregion bei der zweiten Rollausrichtung in dem zweiten Bild;

Messen (450) einer ersten Breite des Handgelenks der Hand bei der ersten Rollausrichtung in dem ersten Bild, um eine erste gemessene Handgelenksbreite zu erhalten, und einer zweiten Breite des Handgelenks der Hand bei der zweiten Rollausrichtung in dem zweiten Bild, um eine zweite gemessene Handgelenksbreite zu erhalten; und

Schätzen (460) einer tatsächlichen Handgelenksbreite der Hand des Benutzers basierend auf der ersten gemessenen Handgelenksbreite, der zweiten gemessenen Handgelenksbreite, des ersten Handgelenkdrehwinkels ($\theta_1$) und des zweiten Handgelenkdrehwinkels ($\theta_2$).

19. Ein nicht flüchtiges, computerlesbares Speichermedium, in dem Anweisungen gespeichert sind, die von einer Computervorrichtung (102) mit einem Prozessor (202) zu implementieren sind, wobei die Anweisungen bei Ausführung durch den Prozessor (202) bewirken, dass die Computervorrichtung (102) zumindest folgende Schritte durchführt:

Auffordern (1010) eines Benutzers, die Hand (704) und das Handgelenk (708) des Benutzers in unmittelbarer Nähe einer Gesichtsregion des Benutzers zu positionieren;

Erhalten (1020) eines Bildes, das die Gesichtsregion und das Handgelenk des Benutzers darstellt;

Messen (1030) eines Pupillenabstands zwischen Augen (702), die in dem Bild dargestellt sind, um einen gemessenen Pupillenabstand zwischen den Augen (702), die in dem Bild dargestellt sind, zu erhalten;

Messen (1040) einer Breite des Handgelenks der Hand, die in dem Bild dargestellt ist, um eine gemessene Handgelenksbreite der Hand, die in dem Bild dargestellt ist, zu erhalten; und

Schätzen (1050) einer tatsächlichen Handgelenksbreite der Hand des Benutzers basierend auf einem tatsächlichen Pupillenabstand, der gemessenen Handgelenksbreite der Hand, die in dem Bild dargestellt ist, und dem gemessenen Pupillenabstand zwischen den Augen (702), die in dem Bild dargestellt sind.

20. Ein nicht flüchtiges, computerlesbares Speichermedium, in dem Anweisungen gespeichert sind, die von einer Computervorrichtung (102) mit einem Prozessor (202) zu implementieren sind, wobei die Anweisungen bei Ausführung durch den Prozessor (202) bewirken, dass die Computervorrichtung (102) zumindest folgende Schritte durchführt:

Auffordern (410) eines Benutzers, die Hand (704) des Benutzers in unmittelbarer Nähe einer Gesichtsregion des Benutzers zu positionieren, wobei die Hand in einer ersten Rollausrichtung gehalten wird und wobei die Hand anschließend in einer zweiten Rollausrichtung gehalten wird;

Erhalten (420) eines ersten Bildes bei der ersten Rollausrichtung und eines zweiten Bildes bei der zweiten Rollausrichtung, wobei das erste Bild und das zweite Bild jeweils/beide die Gesichtsregion und ein Handgelenk (708) des Benutzers darstellen;

Bestimmen (430) eines ersten Handgelenkdrehwinkels ($\theta_1$) bei der ersten Rollausrichtung in dem ersten Bild und eines zweiten Handgelenkdrehwinkels ($\theta_2$) bei der zweiten Rollausrichtung in dem zweiten Bild;

Messen (440) eines ersten Durchmessers einer Iris (706) in der Gesichtsregion bei der ersten Rollausrichtung in dem ersten Bild und eines zweiten Durchmessers der Iris in der Gesichtsregion bei der zweiten Rollausrichtung in dem zweiten Bild;

Messen (450) einer ersten Breite des Handgelenks der Hand bei der ersten Rollausrichtung in dem ersten Bild, um eine erste gemessene Handgelenksbreite zu erhalten, und einer zweiten Breite des Handgelenks der Hand bei der zweiten Rollausrichtung in dem zweiten Bild, um eine zweite gemessene Handgelenksbreite zu erhalten; und

Schätzen (460) einer tatsächlichen Handgelenksbreite der Hand des Benutzers basierend auf der ersten gemessenen Handgelenksbreite, der zweiten gemessenen Handgelenksbreite, des ersten Handgelenkdreh-winkels ($\theta_1$) und des zweiten Handgelenkdrehwinkels ($\theta_2$).

## Revendications

1. Procédé mis en œuvre dans un dispositif informatique (102), comprenant le fait de :

   inviter (310) un utilisateur à placer la main (704) et le poignet (708) de l'utilisateur à proximité étroite d'une région faciale de l'utilisateur ;
   obtenir (320) une image représentant la région faciale et le poignet de l'utilisateur ;
   mesurer (330) un diamètre d'un iris (706) dans la région faciale représentée dans l'image pour obtenir un diamètre mesuré de l'iris dans la région faciale représentée dans l'image ;
   mesurer (340) une largeur du poignet de la main représentée dans l'image pour obtenir une largeur mesurée du poignet de la main représentée dans l'image ; et
   estimer (350) une largeur de poignet réelle de la main de l'utilisateur sur la base d'un diamètre d'iris réel, du diamètre mesuré de l'iris dans la région faciale représentée dans l'image et de la largeur mesurée du poignet de la main représentée dans l'image pour obtenir une largeur de poignet réelle estimée de la main de l'utilisateur.

2. Procédé selon la revendication 1, dans lequel le fait d'estimer (350) la largeur de poignet réelle de la main de l'utilisateur est effectuée en fonction de l'équation suivante :

$$\text{largeur de poignet réelle estimée} = \text{diamètre d'iris réel } x \ \frac{\text{largeur de poignet mesurée}}{\text{diamètre d'iris mesuré}},$$

   dans lequel le diamètre d'iris réel est égal à 11,7, et dans lequel la largeur de poignet réelle estimée de la main de l'utilisateur est mesurée en millimètres.

3. Procédé selon la revendication 1, comprenant en outre le fait de :

   estimer une circonférence de poignet réelle de la main de l'utilisateur sur la base de la largeur de poignet réelle estimée de la main de l'utilisateur pour obtenir une circonférence de poignet réelle estimée ; et
   effectuer une application virtuelle d'un accessoire sur la main sur la base d'au moins une parmi : une circonférence de poignet réelle estimée et la largeur de poignet réelle estimée de la main de l'utilisateur.

4. Procédé selon la revendication 3, dans lequel la circonférence de poignet réelle estimée est estimée sur la base de la largeur de poignet réelle estimée de la main de l'utilisateur en fonction de l'équation suivante :

$$\text{circonférence de poignet réelle estimée} = \text{largeur de poignet réelle estimée } x \ R,$$

   dans lequel R est un rapport statistiquement dérivé entre une valeur de largeur de poignet réelle et la circonférence de poignet réelle.

**5.** Procédé mis en œuvre dans un dispositif informatique (102), comprenant le fait de :

inviter (1010) un utilisateur à placer la main (704) et le poignet (708) de l'utilisateur à proximité étroite d'une région faciale de l'utilisateur ;

obtenir (1020) une image représentant la région faciale et le poignet de l'utilisateur ;

mesurer (1030) une distance pupillaire entre des yeux (702) représentés dans l'image pour obtenir une distance pupillaire mesurée entre les yeux (702) représentés dans l'image ;

mesurer (1040) une largeur du poignet de la main représentée dans l'image pour obtenir une largeur de poignet mesurée de la main représentée dans l'image ; et

estimer (1050) une largeur de poignet réelle de la main de l'utilisateur sur la base d'une distance pupillaire réelle, de la largeur de poignet mesurée de la main représentée dans l'image et de la distance pupillaire mesurée entre les yeux (702) représentés dans l'image pour obtenir une largeur de poignet réelle estimée de la main de l'utilisateur.

**6.** Procédé selon la revendication 5, comprenant en outre le fait de :

estimer une circonférence de poignet réelle de la main de l'utilisateur sur la base de la largeur de poignet réelle estimée de la main de l'utilisateur pour obtenir une circonférence de poignet réelle estimée ; et

effectuer une application virtuelle d'un accessoire sur la main sur la base d'au moins une parmi : la circonférence de poignet réelle estimée, ou la largeur de poignet réelle estimée de la main de l'utilisateur.

**7.** Procédé selon la revendication 5,

dans lequel la largeur de poignet réelle estimée de la main de l'utilisateur est estimée en fonction de l'équation suivante :

$$largeur\ de\ poignet\ réelle\ estimée = distance\ pupillaire\ réelle\ x\ \frac{largeur\ de\ poignet\ mesurée}{distance\ pupillaire\ mesurée},$$

dans lequel la distance pupillaire réelle est spécifiée par l'utilisateur.

**8.** Système, comprenant :

une mémoire (214) stockant des instructions ;

un processeur (202) couplé à la mémoire (214) et configuré par les instructions pour au moins :

inviter (310) un utilisateur à placer la main (704) de l'utilisateur incluant un poignet (708) de l'utilisateur à proximité étroite d'une région faciale de l'utilisateur ;

obtenir (320) une image représentant la région faciale et le poignet de l'utilisateur ;

mesurer (330) un diamètre d'un iris (706) dans la région faciale représentée dans l'image pour obtenir un diamètre mesuré de l'iris dans la région faciale représentée dans l'image ;

mesurer (340) une largeur du poignet de la main représentée dans l'image pour obtenir une largeur mesurée du poignet de la main représentée dans l'image ; et

estimer (350) une largeur de poignet réelle de la main de l'utilisateur sur la base d'un diamètre d'iris réel, du diamètre mesuré de l'iris dans la région faciale représentée dans l'image et de la largeur mesurée du poignet de la main représentée dans l'image pour obtenir une largeur de poignet réelle estimée de la main de l'utilisateur.

**9.** Système selon la revendication 8, dans lequel le processeur (202) est configuré pour estimer une circonférence de poignet réelle de la main de l'utilisateur sur la base de la largeur de poignet réelle estimée de la main de l'utilisateur pour obtenir une circonférence de poignet réelle estimée ; et pour effectuer une application virtuelle d'un accessoire sur la main sur la base d'au moins une parmi : la circonférence de poignet réelle estimée ou la largeur de poignet réelle estimée de la main de l'utilisateur.

**10.** Système selon la revendication 9, dans lequel le processeur (202) est configuré pour estimer la circonférence de poignet réelle estimée sur la base de la largeur de poignet réelle estimée en fonction de l'équation suivante :

$$circonf\acute{e}rence\ de\ poignet\ r\acute{e}elle\ estim\acute{e}e =$$

$$largeur\ de\ poignet\ r\acute{e}elle\ estim\acute{e}e\ x\ R,$$

dans lequel R est un rapport statistiquement dérivé entre une valeur de largeur de poignet réelle et la circonférence de poignet réelle.

11. Système selon la revendication 8, dans lequel le processeur (202) est configuré pour estimer la largeur de poignet de la main de l'utilisateur en fonction de l'équation suivante :

$$largeur\ de\ poignet\ r\acute{e}elle\ estim\acute{e}e =$$
$$diam\grave{e}tre\ d'iris\ r\acute{e}el\ x\ \frac{largeur\ de\ poignet\ mesur\acute{e}e}{diam\grave{e}tre\ d'iris\ mesur\acute{e}},$$

dans lequel le diamètre d'iris réel est égal à 11,7, et dans lequel la largeur de poignet réelle estimée de la main de l'utilisateur est mesurée en millimètres.

12. Support de stockage non transitoire lisible par ordinateur stockant des instructions à mettre en œuvre par un dispositif informatique (102) comportant un processeur (202), dans lequel les instructions, lorsqu'elles sont exécutées par le processeur (202), amènent le dispositif informatique (102) à au moins :

inviter (310) un utilisateur à placer la main (704) de l'utilisateur incluant un poignet (708) de l'utilisateur à proximité étroite d'une région faciale de l'utilisateur ;
obtenir (320) une image représentant la région faciale et le poignet de l'utilisateur ;
mesurer (330) un diamètre d'un iris (706) dans la région faciale représentée dans l'image pour obtenir un diamètre mesuré de l'iris dans la région faciale représentée dans l'image ;
mesurer (340) une largeur du poignet de la main représentée dans l'image pour obtenir une largeur mesurée du poignet de la main représentée dans l'image ; et
estimer (350) une largeur de poignet réelle de la main de l'utilisateur sur la base d'un diamètre d'iris réel, du diamètre mesuré de l'iris dans la région faciale représentée dans l'image et de la largeur mesurée du poignet de la main représentée dans l'image pour obtenir une largeur de poignet réelle estimée de la main de l'utilisateur.

13. Support de stockage non transitoire lisible par ordinateur selon la revendication 12, dans lequel le processeur (202) est en outre configuré pour :

estimer une circonférence de poignet réelle de la main de l'utilisateur sur la base de la largeur de poignet réelle estimée de la main de l'utilisateur pour obtenir une circonférence de poignet réelle estimée de la main de la main de l'utilisateur ; et
effectuer une application virtuelle d'un accessoire sur la main sur la base d'au moins une parmi : la circonférence de poignet réelle estimée et la largeur de poignet réelle estimée de la main de l'utilisateur.

14. Support de stockage non transitoire lisible par ordinateur selon la revendication 13,

dans lequel le processeur (202) est configuré pour estimer la circonférence de poignet réelle estimée sur la base de la largeur de poignet réelle estimée de la main de l'utilisateur en fonction de l'équation suivante :

$$circonf\acute{e}rence\ de\ poignet\ r\acute{e}elle\ estim\acute{e}e =$$

$$largeur\ de\ poignet\ r\acute{e}elle\ estim\acute{e}e\ x\ R,$$

dans lequel R est un rapport statistiquement dérivé entre une valeur de largeur de poignet réelle et la circonférence de poignet réelle.

15. Procédé mis en œuvre dans un dispositif informatique (102), comprenant le fait de :

inviter (410) un utilisateur à placer la main (704) de l'utilisateur à proximité étroite d'une région faciale de l'utilisateur, la main étant maintenue dans une première orientation de roulis et puis la main étant maintenue dans une deuxième orientation de roulis ;

19

obtenir (420) une première image dans la première orientation de roulis et une deuxième image dans la deuxième orientation de roulis, la première image et la deuxième image représentant chacune la région faciale et un poignet (708) de l'utilisateur ;

déterminer (430) un premier angle de rotation de poignet ($\theta_1$) dans la première orientation de roulis dans la première image et un deuxième angle de rotation de poignet ($\theta_2$) dans la deuxième orientation de roulis dans la deuxième image ;

mesurer (440) un premier diamètre d'un iris (706) dans la région faciale dans la première orientation de roulis dans la première image et un deuxième diamètre de l'iris dans la région faciale dans la deuxième orientation de roulis dans la deuxième image ;

mesurer (450) une première largeur du poignet de la main dans la première orientation de roulis dans la première image pour obtenir une première largeur de poignet mesurée et une deuxième largeur du poignet de la main dans la deuxième orientation de roulis dans la deuxième image pour obtenir une deuxième largeur de poignet mesurée ; et

estimer (460) une largeur de poignet réelle de la main de l'utilisateur sur la base de la première largeur de poignet mesurée, de la deuxième largeur de poignet mesurée, du premier angle de rotation de poignet ($\theta_1$) et du deuxième angle de rotation de poignet ($\theta_2$) pour obtenir une largeur de poignet réelle estimée de la main de l'utilisateur.

**16.** Procédé selon la revendication 15, comprenant en outre le fait de :

estimer une circonférence de poignet réelle de la main de l'utilisateur sur la base de la largeur de poignet réelle estimée de la main de l'utilisateur pour obtenir une circonférence de poignet réelle estimée ; et

effectuer une application virtuelle d'un accessoire sur la main sur la base d'au moins une parmi : la circonférence de poignet réelle estimée et la largeur de poignet réelle estimée de la main de l'utilisateur.

**17.** Système, comprenant :

une mémoire (214) stockant des instructions ;
un processeur (202) couplé à la mémoire (214) et configuré par les instructions pour au moins :

inviter (1010) un utilisateur à placer la main (704) et le poignet (708) de l'utilisateur à proximité étroite d'une région faciale de l'utilisateur ;

obtenir (1020) une image représentant la région faciale et le poignet de l'utilisateur ;

mesurer (1030) une distance pupillaire entre les deux (702) représentés dans l'image pour obtenir une distance pupillaire mesurée entre les yeux (702) représentés dans l'image ;

mesurer (1040) une largeur du poignet de la main représentée dans l'image pour obtenir une largeur de poignet mesurée de la main représentée dans l'image ; et

estimer (1050) une largeur de poignet réelle de la main de l'utilisateur sur la base d'une distance pupillaire réelle, de la largeur de poignet mesurée de la main représentée dans l'image et de la distance pupillaire mesurée entre les yeux (702) représentés dans l'image.

**18.** Système, comprenant :

une mémoire (214) stockant des instructions ;
un processeur (202) couplé à la mémoire (214) et configuré par les instructions pour au moins :

inviter (410) un utilisateur à placer la main (704) de l'utilisateur à proximité étroite d'une région faciale de l'utilisateur, la main étant maintenue dans une première orientation de roulis et puis la main étant maintenue dans une deuxième orientation de roulis ;

obtenir (420) une première image dans la première orientation de roulis et une deuxième image dans la deuxième orientation de roulis, la première image et la deuxième image représentant chacune la région faciale et un poignet (708) de l'utilisateur ;

déterminer (430) un premier angle de rotation de poignet ($\theta_1$) dans la première orientation de roulis dans la première image et un deuxième angle de rotation de poignet ($\theta_2$) dans la deuxième orientation de roulis dans la deuxième image ;

mesurer (440) un premier diamètre d'un iris (706) dans la région faciale dans la première orientation de roulis dans la première image et un deuxième diamètre de l'iris dans la région faciale dans la deuxième orientation de roulis dans la deuxième image ;

mesurer (450) une première largeur du poignet de la main dans la première orientation de roulis dans la première image pour obtenir une première largeur de poignet mesurée et une deuxième largeur du poignet de la main dans la deuxième orientation de roulis dans la deuxième image pour obtenir une deuxième largeur de poignet mesurée ; et

estimer (460) une largeur de poignet réelle de la main de l'utilisateur sur la base de la première largeur de poignet mesurée, de la deuxième largeur de poignet mesurée, du premier angle de rotation de poignet ($\theta_1$) et du deuxième angle de rotation de poignet ($\theta_2$).

**19.** Support de stockage non transitoire lisible par ordinateur stockant des instructions à mettre en œuvre par un dispositif informatique (102) comportant un processeur (202), dans lequel les instructions, lorsqu'elles sont exécutées par le processeur (202), amènent le dispositif informatique (102) à au moins :

inviter (1010) un utilisateur à placer la main (704) et le poignet (708) de l'utilisateur à proximité étroite d'une région faciale de l'utilisateur ;

obtenir (1020) une image représentant la région faciale et le poignet de l'utilisateur ;

mesurer (1030) une distance pupillaire entre des yeux (702) représentés dans l'image pour obtenir une distance pupillaire mesurée entre les yeux représentés dans l'image ;

mesurer (1040) une largeur du poignet de la main représentée dans l'image pour obtenir une largeur de poignet mesurée de la main représentée dans l'image ; et

estimer (1050) une largeur de poignet réelle de la main de l'utilisateur sur la base d'une distance pupillaire réelle, de la largeur de poignet mesurée de la main représentée dans l'image et de la distance pupillaire mesurée entre les yeux (702) représentés dans l'image.

**20.** Support de stockage non transitoire lisible par ordinateur stockant des instructions à mettre en œuvre par un dispositif informatique (102) comportant un processeur (202), dans lequel les instructions, lorsqu'elles sont exécutées par le processeur (202), amènent le dispositif informatique (102) à au moins :

inviter (410) un utilisateur à placer la main (704) de l'utilisateur à proximité étroite d'une région faciale de l'utilisateur, la main étant maintenue dans une première orientation de roulis puis la main étant maintenue dans une deuxième orientation de roulis ;

obtenir (420) une première image dans la première orientation de roulis et une deuxième image dans la deuxième orientation de roulis, la première image et la deuxième image représentant chacune la région faciale et un poignet (708) de l'utilisateur ;

déterminer (430) un premier angle de rotation de poignet ($\theta_1$) dans la première orientation de roulis dans la première image et un deuxième angle de rotation de poignet ($\theta_2$) dans la deuxième orientation de roulis dans la deuxième image ;

mesurer (440) un premier diamètre d'un iris (706) dans la région faciale dans la première orientation de roulis dans la première image et un deuxième diamètre de l'iris dans la région faciale dans la deuxième orientation de roulis dans la deuxième image ;

mesurer (450) une première largeur du poignet de la main dans la première orientation de roulis dans la première image pour obtenir une première largeur de poignet mesurée et une deuxième largeur du poignet de la main dans la deuxième orientation de roulis dans la deuxième image pour obtenir une deuxième largeur de poignet mesurée ; et

estimer (460) une largeur de poignet réelle de la main de l'utilisateur sur la base de la première largeur de poignet mesurée, de la deuxième largeur de poignet mesurée, du premier angle de rotation de poignet ($\theta_1$) et du deuxième angle de rotation de poignet ($\theta_2$).

COMPUTING DEVICE  102

CONTACTLESS WRIST SIZER 104

IMPORT MODULE 106

PROXIMITY SENSOR 107

FACIAL REGION TRACKER 108

HAND REGION TRACKER 110

WRIST SIZE ESTIMATOR 112

FIG. 1

PROCESSING DEVICE 202

PERIPHERAL INTERFACE 211

NETWORK INTERFACE 206

DISPLAY 208

DATA BUS 210

MEMORY 214

OPERATING SYSTEM 216

104

106 108
110 112

I/O INTERFACES 204

226

COMPUTING DEVICE 102

FIG. 2

START

300

310 — Prompt a user to place the user's hand and wrist next to a facial region of the user

320 — Obtain an image depicting the facial region and the wrist of the user

330 — Measure a diameter of an iris in the facial region depicted in the image

340 — Measure a width of the wrist of the hand depicted in the image

350 — Estimate an actual wrist width of the user's hand

END

# FIG. 3

400

START

410 — Prompt user regarding placement of hand next to facial region

420 — Obtain an image of a user when trigger event is detected

430 — Determine current wrist rotation angle

440 — Measure a diameter of an iris in the facial region in the image at the current wrist rotation angle

450 — Measure a width of the wrist of the hand depicted in the image at the current wrist rotation angle

N wrist rotation angles

460 — Estimate an actual wrist width of the hand based on the first measured wrist width, the second measured wrist width, the first wrist rotation angle ($\theta_1$), and the second wrist rotation angle ($\theta_2$)

END

# FIG. 4

502

102

**WRIST SIZE ESTIMATOR**

Please bring your hand closer to your face with the palm facing you.

504

# FIG. 5

602

WRIST SIZE ESTIMATOR

Please stay still while an image is
captured.

604

## FIG. 6

Measured iris diameter

Measured wrist width

wrist rotation angle
$(\theta)$

FIG. 7

FIG. 8

902

904

First wrist rotation angle ($\theta_1$)    Second wrist rotation angle ($\theta_2$)

# FIG. 9

START

1000

1010 — Prompt a user to place the user's hand including a wrist next to a facial region of the user

1020 — Obtain an image depicting the facial region and a wrist of the user

1030 — Measure a pupillary distance between eyes depicted of the image

1040 — Measure a width of the wrist of the hand of the image

1050 — Estimate an actual wrist width of the user's hand based an actual pupillary distance, the measured wrist width of the hand of the image and the measured pupillary distance of the hand of the image

END

## FIG. 10

# EP 4 246 470 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- A Multi-Gesture Interaction System Using a 3-D Iris Disk Model for Gaze Estimation and an Active Appearance Model for 3-D Hand Pointing. **MICHAEL J REALE et al.** IEEE Transactions on Multimedia. IEEE, 01 June 2011, vol. 13, 474-486